# FASCICULE DE BREVET EUROPEEN

(11) **EP 1 177 766 B1**
(45) Date de publication et mention de la délivrance du brevet: **13.05.2009**
(21) Numéro de dépôt: 01401938.4
(22) Date de dépôt: 19.07.2001
(51) Int. Cl.: A61B 5/103, A61B 5/00

(54) **Procédé d'acquisition d'une image d'une zone non dermatoglyphique de la peau au moyen d'un dispositif d'acquisition comprenant un capteur non optique.**
Verfahren zur Erfassung von nicht-dermatoglyphischem Hautbereich mittels einer Erfassungsvorrichtung mit einem nicht-optischen Detektor
Method for the acquisition of the image of a non-dermatoglyphic area of the skin with an acquisition device comprising a non-optical sensor

(30) Priorité: 01.08.2000 FR 0010144
(43) Date de publication de la demande: 06.02.2002
(73) Titulaire: L'ORÉAL, 75008 Paris (FR)
(72) Inventeur: Leveque, Jean-Luc, 93340 Le Raincy (FR)
(74) Mandataire: Tanty, François

(56) Documents cités:
- EP-A- 0 748 608
- EP-A- 0 885 587
- EP-A- 0 969 477
- GB-A- 2 044 928
- US-A- 4 066 068
- US-A- 4 849 885
- D.H. Turnbull et al.: "Ultrasound Backscatter Microscope for Skin Imaging", IEEE Ultrasonics Symposium, 1993, pages 985-988

## Description

La présente invention concerne l'observation de la peau humaine ou des cheveux, en vue notamment d'établir un diagnostic et de préconiser, le cas échéant, un soin adapté.

L'invention concerne plus particulièrement, mais non exclusivement, un procédé permettant de déterminer certains paramètres d'une zone non dermatoglyphique de la peau_{.}

L'expression *zone non dermatoglyphique de la peau* doit se comprendre, au sens de la présente invention, comme étant une zone de la peau sensiblement dépourvue de dermatoglyphes, lesquels ne s'altèrent pas avec l'âge. Les dermatoglyphes sont présents aux régions palmaires, notamment aux extrémités des doigts où ils constituent les empreintes digitales. Les zones de la peau dépourvues de dermatoglyphes s'altèrent avec l'âge et présentent des plicatures ayant une origine mécanique, inscrites en creux ou en relief, certaines portant le nom de rides.

Les brevets US 4 353 056 et US 5 864 296 et la demande EP 0 969 477 concernent des détecteurs d'empreintes digitales permettant l'identification d'un utilisateur.

Notamment, dans la demande EP-A-0 969 477 un ensemble de données d'une , zone de la peau du doigt humain ou de la peau du nez d'un chien est acquis au moyen d'un capteur capacitif permettant d'obtenir une information sur le relief microscopique à la surface de ladite zone.

Il existé un besoin pour disposer d'un moyen relativement simple et peu coûteux permettant de renseigner une personne sur l'état de sa peau afin de déterminer si un soin cosmétique est nécessaire.

Un tel moyen se doit d'être relativement simple et peu coûteux pour une utilisation à grande échelle.

Il existe également un besoin pour faciliter le choix d'un soin adapté compte tenu de la nature et/ou de l'état de la peau ou des cheveux d'une personne donnée.

Il existe encore un besoin pour disposer d'un moyen capable de mettre en évidence les premiers effets d'un traitement de la peau ou des cheveux, essentiellement en vue d'encourager la personne faisant l'objet de ce traitement à poursuivre celui-ci.

La demande de brevet britannique GB-A-2 288 511 décrit un système visant à établir un diagnostic à distance et comprenant une caméra optique.

L'image d'une anomalie de la peau est acquise par cette caméra puis envoyée, sous un format numérique, dans un centre de consultation éloigné où un dermatologue peut la consulter et émettre un diagnostic.

Un tel système implique l'intervention d'un médecin.

En outre, son coût est relativement élevé en raison de l'utilisation d'une caméra et aucune application cosmétique de ce système n'est prévue.

L'invention vise notamment à répondre à tout ou partie des besoins mentionnés plus haut

Elle y parvient grâce au nouveau procédé tel que défini dans la revendication 1.

Par *capteur non optique,* on entend au sens de la présente invention un capteur capable de délivrer des informatisons utiles en réponse à une excitation ne faisant pas intervenir la lumière visible et de préférence sans moyens de focalisation tels que des lentilles.

Le capteur non optique peut notamment être un capteur non thermique.

Par *image*, il faut comprendre un ensemble de données et/ou de signaux représentatifs de l'aspect de la zone étudiée.

Par *micro-relief,* on entend le relief microscopique à la surface de la peau ou des cheveux notamment lié à la présence des pligatures telles que les rides, ridules, mais aussi des pores, cellules mortes, poils, écailles, ainsi que le relief lié par exemple à la sécheresse de la peau.

La Demanderesse a constaté de manière inattendue qu'il était possible de tirer d'une image acquise par un capteur non optique des informations utiles pour la détermination de certains paramètres de la peau et/ou pour établir un diagnostic de l'état de la peau et/ou pour mettre en évidence le résultat d'un traitement cosmétique ou autre.

Un avantage, parmi d'autres, de la présente invention réside dans le fait que le coût d'un capteur non optique peut s'avérer nettement inférieur à celui d'un capteur optique et être par conséquent compatible avec une commercialisation à grande échelle, non limitée au secteur professionnel.

La surface active est définie de préférence par une pluralité de cellules de détection élémentaires disposées selon au moins une rangée, et de préférence selon plusieurs rangées juxtaposées.

D'une manière générale, plus la densité surfacique de cellules de détection élémentaires est importante, meilleure sera la résolution.

De préférence, le dispositif d'acquisition est agencé pour délivrer l'image de la zone observée sous forme numérique, ce qui facilite par exemple sa transmission à un micro-ordinateur et/ou sa transmission par réseau, notamment par le réseau Internet.

De préférence également, le dispositif d'acquisition est agencé pour acquérir une image d'une zone suffisamment étendue pour être représentative sur le plan statistique, de surface comprise de préférence entre 0,2 cm² environ et 2 cm² environ et de préférence encore comprise entre 0,25 cm² et 1 cm² environ.

L'acquisition de l'image peut être effectuée par exemple de manière statique, sans déplacement relatif du capteur par rapport à la zone étudiée.

Une telle acquisition est possible en général lorsque la surface active comporte un grand nombre de cellules de détection élémentaires disposées selon plusieurs rangées juxtaposées.

En variante, l'acquisition peut être effectuée de manière dynamique, avec déplacement relatif entre le capteur et la zone étudiée.

Une telle acquisition dynamique est préférée lorsque la surface active se présente sous la forme d'une barrette de cellules de détection élémentaires, le déplacement relatif de la zone étudiée par rapport au capteur s'effectuant alors de préférence perpendiculairement à la direction longitudinale de la barrette.

L'image acquise peut être une image 2D de la zone étudiée, cette image 2D pouvant néanmoins fournir un grand nombre d'informations utiles.

Cependant, en faisant une pluralité d'acquisitions simultanées de la pression de contact du capteur et de limage de la zone étudiée, on peut éventuellement en tirer une information supplémentaire sur la structure du micro-relief de la zone étudiée.

Ainsi, l'image qui est acquise peut être une image 3D de la zone étudiée lorsque le capteur et/ou son environnement le permettent.

L'acquisition de l'image peut être effectuée sans contact du capteur et de la zone étudiée (par exemple grâce à un effet de champ électrique) ou en variante, avec contact du capteur et de la zone étudiée.

Lorsque l'acquisition de l'image s'effectue avec contact de la zone étudiée et du capteur, il est avantageux de mesurer la pression de contact du capteur et de la zone étudiée au cours de l'acquisition de l'image, car la surface de contact entre la zone étudiée et le capteur, donc l'image produite, est susceptible de varier en fonction de la pression de contact.

Lorsque la pression de contact entre la zone étudiée et le capteur n'est pas mesurée, il est préférable que l'acquisition de l'image s'effectue avec une pression de contact sensiblement constante.

Le capteur présente de préférence une résolution spatiale comprise entre 10 et 100 µm, de préférence encore entre 25 et 75 µm, et de préférence encore voisine de 50 µm environ. Le capteur peut notamment présenter une résolution permettant de détecter un relief inférieur ou égal à 100 µm

Cette dernière résolution correspond sensiblement à celle des capteurs les plus répandus ayant une surface active sensible à une charge électrique ou aux variations de température, utilisés pour la reconnaissance des empreintes digitales, et par conséquent susceptibles d'être fabriqués en grand nombre à un coût relativement faible.

De préférence, le traitement qui est effectué est un traitement numérique ne nécessitant pas l'intervention d'un opérateur humain, notamment un médecin.

Un tel traitement est alors susceptible d'être effectué d'une manière entièrement automatique, rapidement et à un coût réduit.

Le traitement de l'image qui est effectué vise par exemple à déterminer une ou plusieurs grandeurs caractéristiques du micro-relief de la peau, afin d'en tirer (éventuellement après recoupement le cas échéant avec des données concernant l'âge, le sexe ou le type ethnique communiquées par la personne concernée) une information sur l'état de la peau et par exemple les concentrations probables dans celle-ci des macromolécules formant la matrice extra-cellulaire du tissu conjonctif, à savoir le collagène, l'élastine, les protéoglycans et les glycoprotéines, et/ou les orientations des faisceaux de collagène par rapport à l'axe du bras, entre autres.

Le traitement de l'image qui est effectué vise avantageusement à fournir une information concernant la densité des lignes de la peau et plus particulièrement le coefficient d'anisotropie de la densité des lignes, c'est-à-dire le rapport de la densité des lignes dans une première direction à la densité des lignes dans une deuxième direction, sensiblement perpendiculaire à la première.

Le traitement effectué sur l'image peut également viser à déterminer le nombre et la taille des pores de la peau, voire la taille et/ou la densité des plateaux délimités par les lignes.

Le traitement effectué sur l'image peut encore permettre de quantifier et/ou de caractériser des rides présentes sur la peau ou de donner une information sur la pilosité.

De préférence, le résultat du traitement effectué sur l'image permet d'établir un diagnostic, voire comporte un diagnostic, ce dernier pouvant tenir compte comme indiqué plus haut de facteurs tels que la densité surfacique des lignes de la peau, le coefficient d'anisotropie de la densité des lignes, la densité surfacique et/ou la taille des pores, la densité et/ou la dimension des plateaux.

Le résultat du traitement permet également, de préférence, de préconiser un soin cosmétique, c'est-à-dire un produit à finalité esthétique pouvant être obtenu sans prescription médicale.

Le traitement de l'image peut être effectué à distance grâce à une transmission de données numériques par réseau, notamment par le réseau Internet.

Le traitement de l'image peut encore être effectué sur place, au moyen d'un micro-ordinateur ou de tout autre moyen de calcul approprié relié au dispositif d'acquisition.

Des images successives dans le temps, de la peau ou des cheveux et/ou des données liées à ces images, peuvent être mémorisées sur un support d'enregistrement.

Des images prises à des moments différents et/ou des données liées à ces images peuvent alors être affichées simultanément ou consécutivement dans un bref intervalle de temps pour permettre à la personne dont la peau est étudiée d'apercevoir les premiers effets d'un traitement ou de constater une dégradation et le besoin d'un traitement.

Une base de données peut être consultée lors du traitement de l'image afin de comparer l'image acquise et/ou des données associées, à des images ou données de comparaison, recueillies auprès d'une large population, dans le but de faciliter l'établissement d'un diagnostic.

La zone de la peau étudiée peut être une région de l'avant-bras ou une région du visage (y compris les lèvres), par exemple les joues.

Le traitement de l'image peut également servir à déterminer un diamètre moyen de cheveu lorsque la zone étudiée est située sur la chevelure.

D'autres caractéristiques et avantages de la présente invention ressortiront à la lecture de la description détaillée qui va suivre, d'exemples non limitatifs de mise en oeuvre de l'invention, et à l'examen du dessin annexé, sur lequel :
- la figure 1 représente de manière schématique un micro-ordinateur relié à un dispositif d'acquisition conforme à un exemple non limitatif de mise en oeuvre de l'invention,
- la figure 2 est une vue analogue à la figure 1, illustrant la connexion du micro-ordinateur à un centre de traitement distant,
- la figure 3 est un schéma en blocs illustrant un agencement possible du dispositif d'acquisition.
- la figure 4 illustre le montage du capteur sur un support sensible à la pression, et
- la figure 5 représente une image de la peau obtenue au moyen d'un capteur non optique.

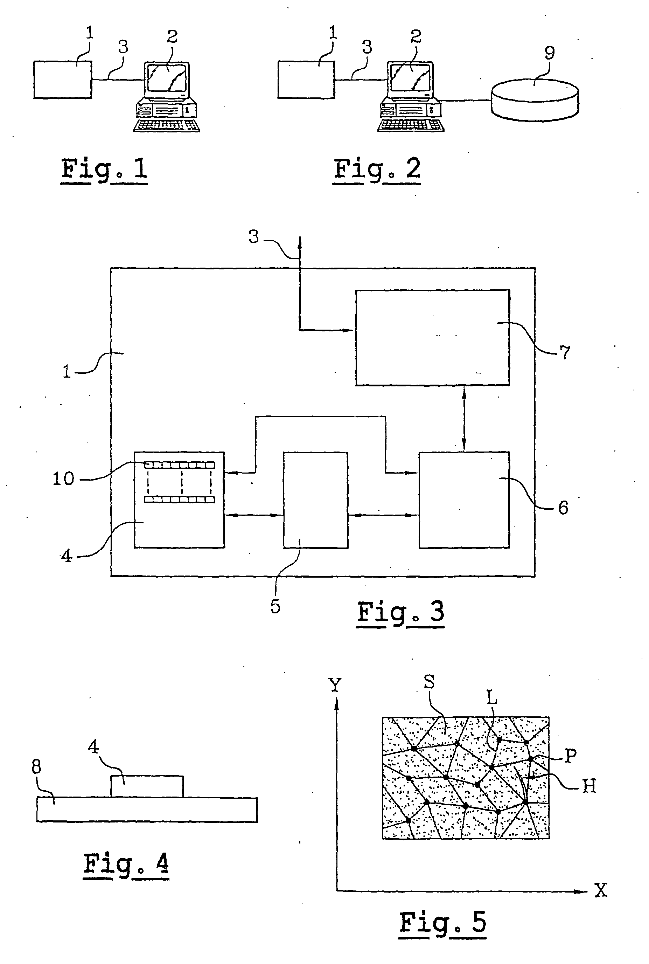

On a représenté sur la figure 1 un dispositif d'acquisition 1 conforme à l'invention, relié à un micro-ordinateur 2 par l'intermédiaire d'un câble 3 de transmission de données.

Le dispositif d'acquisition 1 est de préférence réalisé sous une forme miniaturisée, facilitant son incorporation dans un boîtier de petites dimensions, pouvant être aisément manipulé et amené au contact ou à proximité immédiate de la zone à étudier, située par exemple sur le visage ou l'avant-bras.

Le dispositif d'acquisition 1 peut comporter, comme illustré sur la figure 3, un capteur non optique 4 relié à un convertisseur 5 analogique/digital et à un circuit de commande 6 gérant le fonctionnement du capteur 4.

Le circuit de commande 6 est lui-même relié à un circuit d'interface 7 permettant l'échange de données numériques avec le micro-ordinateur 2.

Le capteur 4 comporte une surface active définie par une pluralité de cellules de détection élémentaires 10 juxtaposées.

Des surfaces actives de ce type sont commercialisées notamment par la société ATMEL sous la dénomination commerciale FINGER CHIP (marque déposée).

Le capteur 4 peut également comporter une surface active définie par une pluralité de cellules de détection élémentaires capacitives, juxtaposées.

Des surfaces actives de ce type sont commercialisées par exemple par la société SGS-THOMSON MICROELECTRONICS sous la dénomination commerciale TOUCH CHIP (marque déposée).

D'autres exemples de capteurs non optiques sont donnés dans les brevets US 4 353 056 et US 5 864 296 notamment.

On a représenté sur la figure 5 une images 2D ou cartographie obtenue au moyen d'une barrette de cellules élémentaires de détection définissant une surface active sensible à la température, autre qu'un capteur capacitif selon la revendication 1, déplacée au contact de l'avant-bras d'une personne

L'homme du métier comprendra à l'examen de cette figure que l'image obtenue permet de renseigner sur l'état de la peau.

On voit en particulier les pores P et les lignes L entourant les plateaux S, ainsi que les poils H.

L'homme du métier comprendra que l'on peut aisément faire subir à une telle image un traitement permettant de déterminer la densité surfacique des lignes L et le coefficient d'anisotropie de la densité, c'est-à-dire le rapport de la densité des lignes L dans une direction X à la densité dans une direction Y perpendiculaire.

Ce rapport évolue avec l'âge et permet de tirer une information sur l'état de vieillissement de la peau, par exemple.

Le traitement de l'image peut aisément être effectué par un logiciel adéquat chargé dans le micro-ordinateur 2, ce logiciel étant en outre avantageusement agencé pour donner un diagnostic et préconiser un produit de soin.

Le micro-ordinateur 2 peut aussi être relié, comme illustré sur la figure 2, au moyen d'un modem à un centre de traitement 9 distant, comprenant un serveur incorporant une base de données ou apte à communiquer avec une base de données.

Le centre de traitement 9 est par exemple accessible, pour l'utilisateur, par un site Internet.

Dans le cas de l'exemple de la figure 2, l'utilisateur transmet l'image de sa peau, obtenue au moyen du dispositif d'acquisition 1, au centre de traitement 9 et ce dernier traite les données reçues de façon à délivrer un diagnostic et éventuellement préconiser un soin.

De préférence, le micro-ordinateur 2 et/ou le centre de traitement 9 sont agencés pour mémoriser des images successivement acquises, afin de permettre à l'utilisateur de percevoir rapidement les effets d'un produit de soin; par exemple.

L'utilisateur peut ainsi être encouragé à poursuivre un traitement dès l'apparition d'un signe d'amélioration, invisible à l'oeil nu ou non décelable par une personne non expérimentée, ou au contraire à changer de traitement lorsque celui-ci se révèle inefficace.

Le micro-ordinateur 2 est avantageusement programmé pour pouvoir afficher plusieurs images de la peau, prises à des moments différents, afin de permettre à l'utilisateur d'effectuer lui-même la comparaison.

Le micro-ordinateur 2 peut également être agencé pour contribuer à mettre en évidence les modifications du micro-relief de la peau entre deux images prises à des moments différents.

Dans les exemples qui viennent d'être décrits, un contact est nécessaire entre la zone étudiée et le capteur lors de l'acquisition de l'image.

Néanmoins, l'invention n'est pas limitée à des capteurs nécessitant un contact avec la zone à observer.

Lorsqu'un contact est nécessaire et que l'image délivrée par le capteur se modifie lorsque la pression exercée par la zone observée sur le capteur change, on mesure avantageusement, comme illustré sur la figure 4, au moyen d'un détecteur approprié 8 sur lequel est monté le capteur 4, la pression de contact pour chaque image afin de tirer de la modification des images avec la pression, une information 3D.

On peut ainsi avec un traitement de données approprié déterminer le profil d'une ride, par exemple.

Bien entendu, l'invention n'est pas limitée aux exemples qui viennent d'être donnés.

## Revendications

1. Procédé comportant les étapes suivantes :
- une image d'une zone non dermatoglyphique de la peau humaine est acquise au moyen d'un dispositif d'acquisition (1) comprenant au moins un capteur (4) non optique permettant d'obtenir une information sur le relief microscopique à la surface de ladite zone, le capteur (4) ayant une surfaces active sensible à au moins une grandeur électrique mesurée grâce à une mesure de capacitance,
- un traitement de l'imagé acquise est effectué et le traitement fournit:
une information concernant les concentrations dans la peau des macromolécules formant la matrice extracellulaire du tissu conjonctif, à savoir le collagène, l'élastine, les protéoglycans et les glycoprotéines, et/ou
une information concernant les orientations des faisceaux de collagène par rapport à l'axe du bras, ou
la densité surfacique des lignes de la peau, et/ou
une information concernant le coefficient d'anisotropie de la densité des lignes de la peau, et/ou
le nombre et la taille des pores de la peau, et/ou
la taille et/ou la densité des plateaux délimités par les lignes de la peau, ou
une information pour quantifier et/ou caractériser des rides ou la pilosité.

2. Procédé selon l'une quelconque des revendications précédentes
**caractérisé par le fait que** la surface active est définie par une pluralité de cellules de détection élémentaires (10) disposées selon au moins une rangée, et de préférence selon plusieurs rangées juxtaposées.

3. Procédé selon la revendication précédente, **caractérisé par le fait que** le dispositif d'acquisition (1) est agencé pour délivrer l'image sous forme numérique.

4. Procédé selon l'une quelconque des revendications précédentes, **caractérisé par le fait que** le dispositif d'acquisition (1) est agencé pour acquérir une image d'une zone de surface comprise entre 0,2 cm² et 2 cm² et de préférence encore comprise entre 0,25 cm² et 1 cm² .

5. Procédé selon l'une quelconque des revendications précédentes, **caractérisé par le fait que** l'acquisition de l'image est effectuée de manière statique, sans déplacement du capteur (4) par rapport à la zone étudiée pendant l'acquisition de l'image.

6. Procédé selon l'une quelconque des revendications 1 à 4, **caractérisé par le fait que** l'acquisition de l'image est effectuée de manière dynamique, avec déplacement relatif entre le capteur et la zone étudiée pendant l'acquisition de l'image.

7. Procédé selon la revendication précédente, **caractérisé par le fait que** la surface active se présente sous la forme d'une barrette de cellules de détection élémentaires.

8. Procédé selon l'une quelconque des revendications précédentes, **caractérisé par le fait que** l'acquisition de l'image est effectuée sans contact du capteur (4) et de la zone étudiée.

9. Procédé selon l'une quelconque des revendications 1 à 7, **caractérisé par le fait que** l'acquisition de l'image est effectuée avec contact du capteur (4) et de la zone étudiée.

10. Procédé selon la revendication précédente, **caractérisé par le fait que** la pression de contact du capteur et de la zone étudiée est mesurée au cours de l'acquisition de l'image.

11. Procédé selon la revendication 9, **caractérisé par le fait que** l'acquisition de l'image s'effectue avec une pression de contact sensiblement constante.

12. Procédé selon l'une quelconque des revendication précédentes, **caractérisé par le fait que** l'image acquise est une image 3D de la zone étudiée.

13. Procédé selon l'une quelconque des revendications 1 à 11, **caractérisé par le fait que** l'image acquise est une image 2D de la zone étudiée.

14. Procédé selon l'une quelconque des revendications précédentes, **caractérisé par le fait que** le capteur présente une résolution spatiale comprise entre 10 et 100 µm**,** de préference entre 25 et 75 µm, et de préférence encore voisine de 50 µm.

15. Procédé selon la revendication 1 **caractérisé par le fait que** le traitement est effectué à distance grâce à une transmission de données numériques par réseau, notamment par le réseau Internet.

16. Procédé selon la revendication 1 , **caractérisé par le fait que** des images successives dans le temps de la zone étudiée, et/ou des données liées à ces images sont mémorisées sur un support d'enregistrement (2;9).

17. Procédé selon l'une quelconque des revendications précédentes, **caractérisé par le fait que** des images prises à des moments différents et/ou des données liées à ces images sont affichées simultanément pour permettre à la personne dont la peau sont etudiés d'apercevoir les effets d'un traitement ou de prendre conscience du besoin d'un traitement.

18. Procédé selon l'une quelconque des revendications précédentes, **caractérisé par le fait que** la zone étudiée est une région de l'avant-bras ou une région du visage.

19. Procédé selon l'une quelconque des revendications précédentes, **caractérisé par le fait que** le dispositif d'acquisition (1) comporte un capteur non-optique ayant une surface active sensible à des variations de capacitance.

20. Procédé selon l'une quelconque des revendications précédentes, **caractérisé par le fait que** le capteur (4) comporte une surface active définie par une pluralité de cellules de détection élémentaires capacitives juxtaposées.

21. Procédé selon l'une quelconque des revendications précédentes, **caractérisé par le fait qu'**il comporte l'étape suivante :
- déterminer la densité des lignes de la peau dans au moins une direction.

22. Procédé selon la revendication précédente, **caractérisé par le fait qu'**il comporte l'étape suivante :
- déterminer une information sur l'état de vieillissement de la peau à partir d'une analyse de la densité des lignes de la peau.

23. Procédé selon l'une quelconque des revendications précédentes,
**caractérisé par le fait qu'**il comporte l'étape suivante :
déterminer l'orientation des faisceaux de collagène par rapport à l'axe d'un bras.

## Claims

1. Method comprising the following steps:
- an image of a non-dermatoglyphic zone of the human skin is acquired by means of an acquisition apparatus (1) comprising at least one non-optical sensor (4) for obtaining information concerning the microrelief at the surface of the said zone, the sensor (4) having an active surface sensitive to at least one electrical magnitude measured by measuring capacitance,
- processing of the acquired image is performed and the processing provides:
information concerning the skin concentrations of macromolecules forming the extracellular matrix of the connective tissue, namely collagen, elastin, proteoglycans and glycoproteins, and/or
information concerning the orientations of the collagen sheaths relative to the axis of the arm, or
the surface density of the skin lines, and/or
information concerning the anisotropy coefficient of the skin line density, and/or
the number and the size of skin pores, and/or
the size and/or the density of the plateaux delimited by the skin lines, or
information for quantifying and/or characterizing the wrinkles or pilosity.

2. Method according to any one of the preceding claims, **characterized in that** the active surface is defined by a plurality of individual detection cells (10) disposed in at least one row, and preferably in a plurality of juxtaposed rows.

3. Method according to the preceding claim, **characterized in that** the acquisition apparatus (1) is arranged to deliver the image in digital form.

4. Method according to any one of the preceding claims, **characterized in that** the acquisition apparatus (1) is arranged to acquire an image of a zone having an area lying in the range 0.2 cm² to 2 cm², and more preferably lying in the range 0.25 cm² to 1 cm².

5. Method according to any one of the preceding claims, **characterized in that** image acquisition is performed statically, without moving the sensor (4) relative to the zone under study during image acquisition.

6. Method according to any one of Claims 1 to 4, **characterized in that** image acquisition is performed dynamically, with relative movement between the sensor and the zone under study during image acquisition.

7. Method according to the preceding claim, **characterized in that** the active surface is in the form of a strip of individual detection cells.

8. Method according to any one of the preceding claims, **characterized in that** image acquisition is performed without the sensor (4) coming into contact with the zone under study.

9. Method according to any one of Claims 1 to 7, **characterized in that** image acquisition is performed with the sensor (4) in contact with the zone under study.

10. Method according to the preceding claim, **characterized in that** the pressure of contact between the sensor and the zone under study is measured during image acquisition.

11. Method according to Claim 9, **characterized in that** image acquisition is performed at substantially constant contact pressure.

12. Method according to any one of the preceding claims, **characterized in that** the acquired image is a 3D image of the zone under study.

13. Method according to any one of Claims 1 to 11, **characterized in that** the acquired image is a 2D image of the zone under study.

14. Method according to any one of the preceding claims, **characterized in that** the sensor presents spatial resolution lying in the range 10 µm to 100 µm, preferably in the range 25 µm to 75 µm, and more preferably close to 50 µm.

15. Method according to Claim 1, **characterized in that** the processing is performed remotely by transmitting digital data over a network, in particular the Internet.

16. Method according to Claim 1, **characterized in that** images of the zone under study that succeed one another in time and/or data associated with said images are stored on a recording medium (2; i 9).

17. Method according to any one of the preceding claims, **characterized in that** images taken at different times and/or data associated with said images are displayed simultaneously to enable the person whose skin is under study to see the effects of treatment or to become aware of the need for treatment.

18. Method according to any one of the preceding claims, **characterized in that** the zone under study is a region of the forearm or a region of the face.

19. Method according to any one of the preceding claims, **characterized in that** the acquisition apparatus (1) comprises a non-optical sensor having an active surface sensitive to variations in capacitance.

20. Method according to any one of the preceding claims, **characterized in that** the sensor (4) comprises an active surface defined by a plurality of juxtaposed capacitive individual detection cells.

21. Method according to any one of the preceding claims, **characterized in that** it comprises the following step:
- determining the density of the skin lines in at least one direction.

22. Method according to the preceding claim, **characterized in that** it comprises the following step:
- determining information concerning the state of skin ageing from analysis of the density of the skin lines.

23. Method according to any one of the preceding claims, **characterized in that** it comprises the following step:
- determining the orientation of the collagen sheaths relative to the axis of an arm.

## Patentansprüche

1. Verfahren, umfassend die folgenden Schritte:
- ein Bild einer nicht dermatoglyphischen Zone der menschlichen Haut wird mit Hilfe einer Erfassungsvorrichtung (1) erfasst, die mindestens einen nicht optischen Fühler (4) umfasst, der es gestattet, eine Information über das mikroskopische Relief an der Oberfläche dieser Zone zu erhalten, wobei der Fühler (4) eine aktive Oberfläche besitzt, die für mindestens eine mit Hilfe einer Kapazitanzmessung gemessene elektrische Größe empfindlich ist,
- eine Behandlung des erfassten Bilds wird vorgenommen und die Behandlung liefert: eine Information betreffend die Konzentrationen der die extrazelluläre Matrix des Bindegewebes bildenden Makromoleküle in der Haut, und zwar von Collagen, Elastin, Proteoglycanen und Glycoproteinen, und/oder
eine Information betreffend die Ausrichtungen der Collagenbündel bezüglich der Achse des Arms oder
die Oberflächendichte der Linien der Haut und/oder
eine Information betreffend den Anisotropiekoeffizient der Dichte der Linien der Haut und/oder
die Anzahl und Größe der Poren der Haut und/oder
die Größe und/oder die Dichte der von den Linien der Haut begrenzten Plateaus oder
eine Information zur Quantifizierung und/oder Charakterisierung der Falten oder der Behaarung.

2. Verfahren nach einem der vorhergehenden Ansprüche, **dadurch gekennzeichnet, dass** die aktive Oberfläche von einer Mehrzahl von Einzelerfassungszellen (10) gebildet ist, die in mindestens einer Reihe und vorzugsweise in mehreren nebeneinander gesetzten Reihen angeordnet sind.

3. Verfahren nach dem vorhergehenden Anspruch, **dadurch gekennzeichnet, dass** die Erfassungsvorrichtung (1) ausgebildet ist, um das Bild in digitaler Form zu liefern.

4. Verfahren nach einem der vorhergehenden Ansprüche, **dadurch gekennzeichnet, dass** die Erfassungsvorrichtung (1) ausgebildet ist, um ein Bild einer Zone mit einer Fläche zwischen 0,2 cm² und 2 cm² und vorzugsweise zwischen 0,25 cm² und 1 cm² zu erfassen.

5. Verfahren nach einem der vorhergehenden Ansprüche, **dadurch gekennzeichnet, dass** die Erfassung des Bilds statisch ohne Bewegung des Fühlers (4) bezüglich der untersuchten Zone während der Erfassung des Bilds durchgeführt wird.

6. Verfahren nach einem der Ansprüche 1 bis 4, **dadurch gekennzeichnet, dass** die Erfassung des Bilds dynamisch mit relativer Bewegung zwischen dem Fühler und der untersuchten Zone während der Erfassung des Bilds durchgeführt wird.

7. Verfahren nach dem vorhergehenden Anspruch, **dadurch gekennzeichnet, dass** die aktive Oberfläche in der Form einer Leiste von Einzelerfassungszellen vorliegt.

8. Verfahren nach einem der vorhergehenden Ansprüche, **dadurch gekennzeichnet, dass** die Erfassung des Bilds ohne Kontakt des Fühlers (4) und der untersuchten Zone vorgenommen wird.

9. Verfahren nach einem der Ansprüche 1 bis 7, **dadurch gekennzeichnet**, die Erfassung des Bilds mit Kontakt des Fühlers (4) und der untersuchten Zone vorgenommen wird.

10. Verfahren nach dem vorhergehenden Anspruch, **dadurch gekennzeichnet, dass** der Kontaktdruck des Fühlers und der untersuchten Zone während der Erfassung des Bilds gemessen wird.

11. Verfahren nach Anspruch 9, **dadurch gekennzeichnet, dass** die Erfassung des Bilds mit einem im Wesentlichen konstanten Kontaktdruck vorgenommen wird.

12. Verfahren nach einem der vorhergehenden Ansprüche, **dadurch gekennzeichnet, dass** das erfasste Bild ein 3D-Bild der untersuchten Zone ist.

13. Verfahren nach einem der Ansprüche 1 bis 11, **dadurch gekennzeichnet, dass** das erfasste Bild ein 2D-Bild der untersuchten Zone ist.

14. Verfahren nach einem der vorhergehenden Ansprüche, **dadurch gekennzeichnet, dass** der Fühler eine räumliche Auflösung zwischen 10 und 100 µm, vorzugsweise zwischen 25 und 75 µm und vorzugsweise noch nahe 50 µm aufweist.

15. Verfahren nach Anspruch 1, **dadurch gekennzeichnet, dass** die Behandlung mit Hilfe einer Übertragung von digitalen Daten über das Netz, insbesondere über das Internet, femdurchgeführt wird.

16. Verfahren nach Anspruch 1, **dadurch gekennzeichnet, dass** zeitlich aufeinanderfolgende Bilder der untersuchten Zone und/oder mit diesen Bildern verbundene Daten auf einem Aufzeichnungsträger (2; 9) gespeichert werden.

17. Verfahren nach einem der vorhergehenden Ansprüche, **dadurch gekennzeichnet, dass** zu verschiedenen Zeitpunkten aufgenommene Bilder und/oder mit diesen Bildern verbundene Daten gleichzeitig angezeigt werden, µm der Person, deren Haut untersucht wird, zu gestatten, die Wirkungen einer Behandlung festzustellen oder die Notwendigkeit einer Behandlung zu erkennen.

18. Verfahren nach einem der vorhergehenden Ansprüche, **dadurch gekennzeichnet, dass** die untersuchte Zone ein Bereich des Unterarms oder ein Bereich des Gesichts ist.

19. Verfahren nach einem der vorhergehenden Ansprüche, **dadurch gekennzeichnet, dass** die Erfassungsvorrichtung (1) einen nicht optischen Fühler mit einer für Kapazitanzänderungen empfindlichen aktiven Oberfläche umfasst.

20. Verfahren nach einem der vorhergehenden Ansprüche, **dadurch gekennzeichnet, dass** der Fühler (4) eine aktive Oberfläche umfasst, die von einer Mehrzahl von nebeneinander gesetzten kapazitiven Einzelerfassungszellen gebildet ist.

21. Verfahren nach einem der vorhergehenden Ansprüche, **dadurch gekennzeichnet, dass** es den folgenden Schritt umfasst:
- die Dichte der Linien der Haut in mindestens einer Richtung bestimmen.

22. Verfahren nach dem vorhergehenden Anspruch, **dadurch gekennzeichnet, dass** es den folgenden Schritt umfasst:
- eine Information über den Alterungszustand der Haut ausgehend von einer Analyse der Dichte der Linien der Haut bestimmen.

23. Verfahren nach einem der vorhergehenden Ansprüche, **dadurch gekennzeichnet, dass** es den folgenden Schritt umfasst:
- die Ausrichtung der Collagenbündel bezüglich der Achse eines Arms bestimmen.
